Europäisches Patentamt

European Patent Office

Office européen des brevets

(11) Publication number: **0 091 153**
**A2**

(12) **EUROPEAN PATENT APPLICATION**

(21) Application number: **83200412.1**

(22) Date of filing: **24.03.83**

(51) Int. Cl.³: **A 41 B 13/02**

(30) Priority: **05.04.82 US 365429**

(43) Date of publication of application:
**12.10.83 Bulletin 83/41**

(84) Designated Contracting States:
**AT BE CH DE FR GB IT LI LU NL SE**

(71) Applicant: **THE PROCTER & GAMBLE COMPANY**
**301 East Sixth Street**
**Cincinnati Ohio 45202(US)**

(72) Inventor: **Foreman, John H.**
**4447 Woodtrail Lane**
**Cincinnati Ohio 45239(US)**

(74) Representative: **Gibson, Tony Nicholas et al,**
**Procter & Gamble (NTC) Limited Whitley Road**
**Longbenton Newcastle upon Tyne NE12 9TS(GB)**

(54) Disposable diaper having a reinforced waist portion.

(57) A disposable diaper is disclosed which exhibits reduced topsheet tearing. The disposable diaper has a reinforcing element positioned between the topsheet and the backsheet and a taping system which utilizes a fastening tape having a topsheet leg which overlays the topsheet. The topsheet leg is affixed to the reinforcing element which has a greater tensile strength and a lower modulus of elasticity than the topsheet.

Fig. 3

Croydon Printing Company Ltd.

EP 0 091 153 A2

# DISPOSABLE DIAPER HAVING
## A REINFORCED WAIST PORTION

John H. Foreman

## Background of the Invention

This invention relates to disposable diapers and the like. In particular, this invention relates to a disposable diaper having a topsheet characterized by a tensile strength lower than that of an underlying reinforcing element.

Infants and incontinent adults are usually clothed in a diaper or diaper-like garment which is capable of absorbing and containing human waste products. Diapers are garments which are drawn up between the legs and fastened about the waist of the wearer and are well known. Such garments may be manufactured from reusable woven cloth materials and worn in conjunction with a fluid impermeable outer garment. In recent years, however, disposable diapers have come into widespread use and have gained a high degree of acceptance as a consumer product. Disposable diapers are designed to combine a fluid impermeable outer garment with a disposable absorbent core forming an integral structure which is intended to be discarded after a single use.

A major in-use problem encountered with disposable diapers is tearing of the topsheet as the diaper is being applied to the infant. Topsheet tearing generally occurs because of the high tensile forces induced in the topsheet as the mother pulls on the fastening tape to get a suitable fit during initial application of the diaper to an infant.

- 2 -

0091153

Many developments in the disposable diaper art have been directed toward improving the characteristics of the topsheet. Some of these developments have resulted in topsheet materials which while having some improved characteristics exhibit reduced tensile strength. When such topsheets are used with a taping system having a topsheet leg affixed to the topsheet, the diaper will experience tearing of the topsheet as the diaper is applied to the infant.

The disposable diapers of the prior art lack the aspects of the present invention whereby the disposable diaper exhibits reduced topsheet tearing. It is therefore an object of the present invention to provide a disposable diaper having a reinforced waist portion.

A further object of the present invention is provided a disposable diaper having a reinforcing element interposed between the topsheet and the backsheet.

A still further object of the present invention is to provide a disposable diaper in which the topsheet legs of the fastening tapes are affixed to the end portions of a reinforcing element.

These and other objects of the present invention will become more fully apparent in the following description of the embodiments of this invention and from the appended claims.

Summary of the Invention

According to the present invention, a disposable diaper is manufactured having a taping system in which one leg of the fastening tapes overlays the topsheet. The disposable diaper is provided with a reinforcing element which resists tensile forces induced in the diaper as the diaper is being applied to the wearer.

The reinforcing element comprises a sheet of polyethylene material positioned between the topsheet and the backsheet. The topsheet leg of the fastening tape is affixed to the reinforcing element through the topsheet. This may be conveniently done by using a suitable adhesive which penetrates the thickness of the topsheet material thereby bonding the topsheet leg of the fastening tape, the topsheet material, and the reinforcing element together.

As the disposable diaper is applied to the wearer, tensile forces are developed along the transverse direction of the disposable diaper at the back waist portion. These tensile forces are transmitted through the tapes to the reinforcing element. The reinforcing element has a strength higher than that of the topsheet and is capable of resisting the tape induced forces. Further, the reinforcing element is less elastic than the topsheet and therefore will bear the tensile forces to a larger extent than the topsheet. Accordingly, the load is carried by the reinforcing element rather than by the topsheet and tearing of the topsheet is thus prevented.

Description of the Drawings

Figure 1 is a perspective view of a disposable diaper which incorporates the present invention and which is Z-folded and ready to be placed on an infant.

Figure 2 is a partially cut-away perspective view of the disposable diaper of Figure 1 prior to being Z-folded.

Figure 3 is a cross-sectional view of the diaper of Figure 2 taken along line 3-3.

Description of a Preferred Embodiment

Referring now to the figures, there is shown a preferred embodiment of the present invention as it would be used in a disposable diaper. As used herein, the term "disposable diaper" refers to a garment generally worn by infants and incontinent persons, which is drawn up between the legs and fastened about the waist of the wearer, and further which garment is intended to be discarded after a single use (i.e., it is not intended to be laundered or otherwise restored and reused).

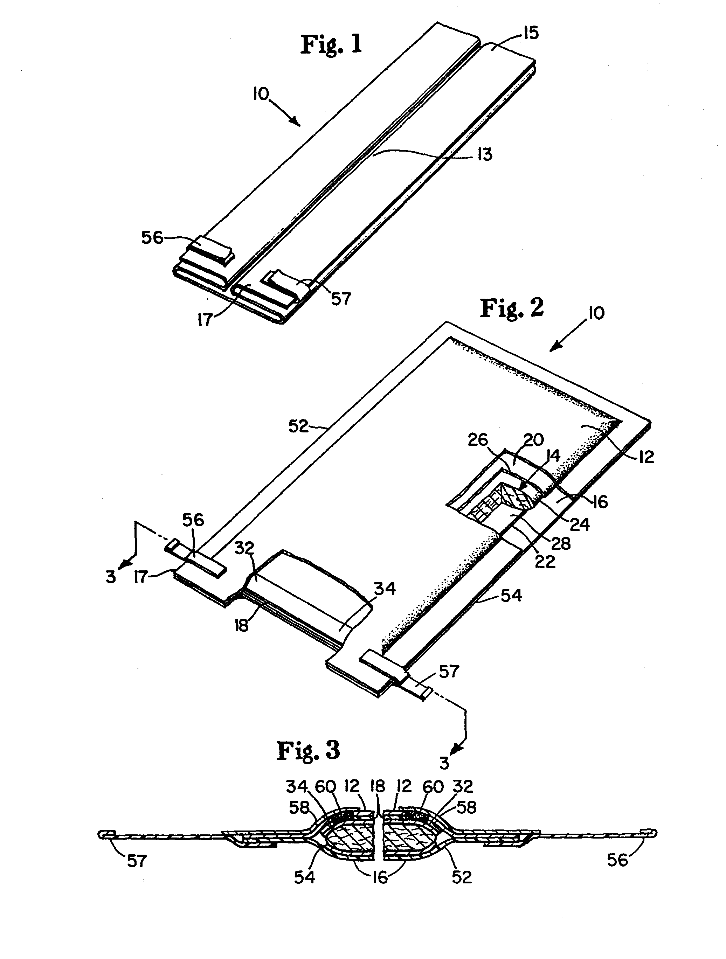

Figure 1 is a perspective view of a disposable diaper 10 having a Z-folded configuration and incorporating the features of the present invention. As shown in Figure 1, the diaper 10 is in condition for placement on a wearer. In general, the crotch portion 13 of the diaper 10 is placed between the wearer's legs and the front and back waist portions 15 and 17 respectively, are joined together by first fastening tape 56 and by second fastening tape 57 so as to encircle the wearer's waist and to hold the diaper 10 in place. While the present invention will be described with reference to a diaper having a Z-folded configuration, it should be understood that diaper configurations and constructions other than those specifically described, such as C-folded and unfolded configurations, may also incorporate the features of the present invention. The terms "Z-folded", "C-folded", and unfolded refer to the configuration of the longitudinal sides 52 and 54 of the diaper 10.

To simplify the description of the present invention the diaper 10 of Figure 1 is shown in Figure 2 in a partially cut-away perspective view prior to its being Z-folded and placed on a wearer. As seen in Figure 2, a preferred disposable diaper 10 basically comprises a liquid permeable topsheet 12, an absorbent core 14, a liquid impermeable backsheet 16, and a reinforcing element 18. While the topsheet 12, absorbent core 14, and backsheet 16 may be assembled in a variety of well known configurations such as are described generally in U.S. Patent Re. 26,151 entitled "Diaper" which issued to R. C. Duncan et al. on January 31, 1967, and in U.S. Patent 3,860,003 entitled "Contractable Side Portions For Disposable Diaper" which issued to K. B. Buell on January 14, 1975, a preferred construction of diaper 10 will now be described.

As can be seen in Figure 2, the topsheet 12 and the backsheet 16 are joined together so as to encase and contain the absorbent core 14. Any suitable means, as hereinafter described, may be used to join the topsheet 12 and the backsheet 16.

The absorbent core 14 may be any means which is generally compressible, conformable, nonirritating to the wearer's skin, and which is capable of absorbing and retaining liquids. A preferred absorbent core 14 has first and second opposed faces 20 and 22 respectively, and comprises an absorbent layer 24 and first and second tissue layers 26 and 28 respectively. The first and second tissue layers 26 and 28 overlay the major surfaces of the absorbent layer 24 to form first and second opposed faces 20 and 22.

The absorbent layer 24 may be manufactured in a wide variety of sizes and shapes (e.g., rectangular, hourglass, etc.) and from a wide variety of liquid absorbent materials commonly used in disposable absorbent articles, such as comminuted wood pulp which is generally referred to as absorbent fluff. Other liquid absorbing materials can also be used for the absorbent layer 24, such as a multiplicity of plies of creped cellulose wadding, absorbent foams or sponges, or any equivalent material. Further, the size and absorbent capacity of the absorbent layer 24 may be varied to accommodate wearers ranging from infants to adults. The preferred embodiment illustrated in Figure 2 has a rectangular absorbent layer 24 and is intended for infants of from 12 pounds to 23 pounds ( 5 kilograms to 10 kilograms). The absorbent layer is, therefore, absorbent fluff approximately 12 inches (31.8 centimeters) wide by 16 inches (40.6 centimeters) long having an absorbent capacity of from 8 to 16 grams of water per gram of absorbent. Accordingly, the absorbent fluff used in the preferred embodiment shown in Figures 2 and 3 weighs approximately from 30 to 56 grams. It should be understood, however, that the size, shape, and total absorbent capacity of the absorbent core 14 may be varied to accommodate wearers ranging from infants to adults. Therefore, other dimensions and even other shapes (e.g., hourglass) may also be used for the absorbent core 14.

The tissue layers 26 and 28 improve the tensile strength of the absorbent core 14 and reduce the tendency of the absorbent layer 24 to split, lump, or ball when wetted. The tissue layers 26 and 28 also help to improve lateral wicking of absorbent liquids, thereby providing a more even distribution of liquids throughout the absorbent core 14. While a number of

- 7 -

0091153

materials and manufacturing techniques may be used to manufacture the tissue layers 26 and 28, satisfactory results have been obtained with sheets of tissue paper having a basis weight of from 10 pounds per 3,000 square feet (16 grams per square meter) and having an air permeability of 100 cubic feet per minute per square foot (30.5 cubic meters per minute per square meter) over a one-half inch (12.8 millimeters) water pressure drop. While the tissue layers 26 and 28 are preferably coterminous with the absorbent layer 24, they may have different dimensions, a different configuration, or they may be omitted entirely.

The second tissue layer 28 is superimposed on the backsheet 16 and is preferably attached thereto by attachment means (not shown) such as those well known in the art. For example, the absorbent core 14 can be secured to the backsheet by a uniform continuous layer of adhesive, a patterned layer of adhesive, or an array of separate lines or spots of adhesive. An adhesive which has been found to be satisfactory is manufactured by Eastman Chemical Products Company of Kingsport, Tennessee and marketed under the tradename Eastobond A3.

The backsheet 16 is impervious to liquids and is preferably manufactured from a thin plastic film, although other flexible liquid impervious materials may also be used. The backsheet 16 prevents the liquids contained in the absorbent core from wetting articles which contact the diaper, such as bedsheets and undergarments. Polyethylene films having a thickness of from 0.0005 to 0.002 inches (0.0012 to 0.0051 centimeters) have been used for the backsheet 16 in a preferred embodiment with satisfactory results.

As used herein, the term "flexible" refers to materials which are compliant and which readily conform to the general shape and contours of the human body. A suitable polyethylene film is manufactured by Monsanto Chemical Company and marketed in the trade as film number 8020.

The topsheet 12 is compliant, soft feeling, and nonirritating to the wearer's skin. Further, the topsheet 12 is liquid permeable permitting liquids to readily penetrate through its thickness. A suitable topsheet 12 may be manufactured from a wide range of materials such as porous foams, apertured plastic films, natural fibers (e.g., wood or cotton fibers), synthetic fibers (e.g., polyester or polypropylene fibers) or from a combination of natural and synthetic fibers and prevents the wearer of the diaper from contacting the absorbent core 14.

A particularly preferred topsheet 12 comprises by weight 65 percent staple length polyester fibers having a denier of 1.5, such as Kodel type 411 polyester fibers marketed by Tennessee Eastman Corporation of Kingsport, Tennessee; 15 percent staple length crimped rayon fibers having a denier of 1.5; and 20 percent acrylic copolymer binder such as Celanese CPE 8335 marketed by Celanese Corporation of Charlotte, North Carolina. As used herein, the term "staple length fibers" refers to those fibers having a length of at least 5/8 inches (15.9 millimeters). Clearly, there are a number of manufacturing techniques which may be utilized to manufacture the topsheet 12. For example, the topsheet 12 may be woven, non-woven, spunbonded, carded or the like. A preferred topsheet 12 is carded, saturated with a binder solution, dried and cured by means well known to those skilled in the art. Preferably, the topsheet 12 has a basis weight range of from 18 to 22 grams per square yard. A preferred topsheet 12 is further characterized by a minimum wet tensile strength of at least 400 grams per centimeter in the machine direction of the topsheet material and at least 55 grams per centimeter in the cross-machine direction of the topsheet material.

Referring now to Figures 2 and 3, it can be seen that the reinforcing element 18 is preferably a continuous strip of flexible material interposed between the topsheet 12 and the backsheet 16 at the back waist portion 17. The reinforcing element 18 has a first end portion 32 disposed toward first longitudinal side 52 and a second end portion 34 disposed toward second longitudinal side 54. Further, the reinforcing element 18 is positioned and shaped so that when the first and second fastening tapes 56 and 57 are affixed to the diaper 10 as hereinafter described, the first and second fastening tapes 56 and 57 are affixed to first and second end portions 32 and 34, respectively. The reinforcing element 18 has a transverse tensile strength greater than that of the topsheet 12 and preferably at least 635 grams per centimeter. In addition, the modulus of elasticity of the reinforcing element 18 is less than the modulus of elasticity of the topsheet 12. Thus, the reinforcing element 18 is chosen so that it will bear the loads induced in the diaper 10 when the tapes 56 are being pulled.

It is, of course, possible to manufacture the reinforcing element from a variety of materials. In a preferred embodiment, the polyethylene film having a thickness of 0.001 inches (0.0025 centimeters) and a transverse tensile strength of 3,550 grams per centimeter was used.

The backsheet 16 is superimposed on the second opposed face 22 of the absorbent core 14 and has dimensions generally larger than those of the absorbent core 14. The topsheet 12 is superimposed on the first opposed face 20 of the absorbent core 14 and is coterminous with the backsheet 16. The backsheet 16 is affixed about its periphery to the topsheet 12 in any suitable manner such as by the use of adhesives. A suitable adhesive is manufactured by National Starch Corporation of Bridgewater, New Jersey and marketed under the tradename "Instant Lock 34-2933", although other adhesives as are well known may also be used. The absorbent core 14 is, therefore, encased between the topsheet 12 and the backsheet 16.

The first and second fastening tapes 56 and 57 are positioned at the back waist portion 17 of the diaper 10 and are placed along first and second longitudinal sides 52 and 54 respectively. The first and second fastening tapes 56 and 57 may be of any of the well known configurations in which the first and second fastening tapes 56 and 57 have a topsheet leg 58. As used herein, the term "topsheet leg" refers to that portion to the first and second fastening tapes 56 and 57 which overlays to topsheet 12.

A suitable fastening tape is disclosed in U.S. Patent 3,848,594 entitled "Tape Fastening System For Disposable Diaper" which issued to K. B. Buell on November 19, 1974.

As can best be seen in Figure 3 the topsheet leg 58 overlays the topsheet 12 and affixment means 60 joins the topsheet leg 58 to the reinforcing element 18 at first end portion 32 and at second end portion 34. Affixment means 60 may be manufactured by any of a variety of well known methods. For example, the topsheet leg 58 may be heat sealed or ultrasonically bonded to the reinforcing element 18. In a preferred embodiment, however, the topsheet leg 58 is affixed to the reinforcing element 18 by gluing. In particular, glue is applied to the topsheet 12 overlying the first and second end portions 32 and 34 respectively. The glue penetrates through the thickness of the topsheet 12 so that when the topsheet 12 is brought into contact with the reinforcing element 18 and with the topsheet leg 58, these elements are joined together.

In use, the diaper 10 is applied to and worn by a wearer in the conventional manner. During the diapering operation, tensile forces are applied through the first and second fastening tapes 56 and 57 to the back waist portion 17. These forces are transmitted to and resisted by the reinforcing element 18, thereby reducing the incidence of tearing in the weaker topsheet 12.

CLAIMS

1.   A disposable diaper comprising:

an absorbent core means (14,24,26,28) for absorbing liquids, said absorbent core means having first and second opposed faces (20,22);

a liquid impervious backsheet (16), said backsheet being superimposed on said second opposed face (22) of said absorbent core means (14);

a liquid permeable topsheet (12), said topsheet being superimposed on said first opposed face (20) of said absorbent core means (14) and said topsheet (12) being affixed to said backsheet (16), said absorbent core means (14) being encased between said topsheet (12) and said backsheet (16);

characterised in that said disposable diaper includes

a reinforcing element (18), said reinforcing element being positioned between said topsheet (12) and said backsheet (16) and having a first end portion (32) and a second end portion (34), said reinforcing element (18) having a transverse tensile strength greater than that of said topsheet (12) and having a modulus of elasticity less than that of said topsheet; and

first and second fastening tapes (56,57), each having a topsheet leg (58), each said topsheet leg overlaying said topsheet and being affixed to said reinforcing element in the area of said end portions (32,34).

2.   A disposable diaper according to Claim 1 wherein said topsheet leg (58), said topsheet (12) and said reinforcing element (18) are glued together.

3.   A disposable diaper according to Claim 1 wherein said topsheet leg (58), said topsheet (12) and said reinforcing element (18) are ultrasonically bonded together.

4.   A disposable diaper according to any one of Claims 1-3 wherein said reinforcing element (18) has a minimum tensile strength of at least 635 grams per centimeter.

1/1

**Fig. 1**

**Fig. 2**

**Fig. 3**